# EUROPEAN PATENT SPECIFICATION

(11) **EP 1 157 158 B1**
(45) Date of publication and mention of the grant of the patent: **14.05.2003**
(21) Application number: 00903905.8
(22) Date of filing: 21.02.2000
(51) Int. Cl.: D06M 16/00, D06M 15/17, D06M 15/03, D06M 11/83, A61L 15/46, A01N 59/16, D21H 21/36

(54) **PROCESS FOR MAKING SUBSTRATES WITH BIOCIDAL PROPERTIES**
VERFAHREN ZUR HERSTELLUNG EINES SUBSTRATS MIT BIOZIDEIGENSCHAFTEN
PROCEDE DE FABRICATION DE SUBSTRATS AYANT DES PROPRIETES BIOCIDES

(30) Priority: 20.02.1999 GB 9903842
(43) Date of publication of application: 28.11.2001
(73) Proprietor: Foxwood Research Limited, Mansfield, Nottinghamshire NG20 8AQ (GB)
(72) Inventor: BLOWES, Phillip Charles, Timberidge, Rickmansworth WD3 4JD (GB); TAYLOR, Alan John, Chesterfield, Derbyshire S43 3HA (GB); ROBERTS, George Nicolas and Walters, Mansfield, Nottinghamshire NG20 8AQ (GB); WOOD, Fran Nicolas and Walters, Mansfield, Nottinghamshire NG20 8AQ (GB)
(74) Representative: Griffin, Kenneth David
(86) International application number: GB0000604
(87) International publication number: WO00049219

(56) References cited:
- EP-A- 0 291 587
- US-A- 5 643 971
- DATABASE WPI Section Ch, Week 199549 Derwent Publications Ltd., London, GB; Class D22, AN 1995-378809 XP002138834 & JP 07 256025 A (NAKAMURA K), 9 October 1995 (1995-10-09)
- DATABASE WPI Section Ch, Week 199651 Derwent Publications Ltd., London, GB; Class A60, AN 1996-514846 XP002138835 & JP 08 268821 A (SANGI KK), 15 October 1996 (1996-10-15)
- DATABASE WPI Section Ch, Week 199641 Derwent Publications Ltd., London, GB; Class D22, AN 1996-406189 XP002138836 & JP 08 196461 A (NAKAMURA K), 6 August 1996 (1996-08-06)
- DATABASE WPI Section Ch, Week 199629 Derwent Publications Ltd., London, GB; Class C07, AN 1996-283363 XP002138837 & JP 08 119805 A (NAKAMURA K), 14 May 1996 (1996-05-14)
- DATABASE WPI Section Ch, Week 198045 Derwent Publications Ltd., London, GB; Class A60, AN 1980-79570C XP002138838 & JP 55 122556 A (NIPPON TENNEN GAS K), 20 September 1980 (1980-09-20)

## Description

This invention relates to a treatment process suitable for application to fibre, yarn, non-woven, woven, knitted fabric, garments, or paper products, hereinafter referred to as 'The Substrate'. It also relates to articles fabricated from these materials, in particular those widely used in the health care and associated industries. The treatment process can be applied to any natural or synthetic substrate or blends therefrom. Typically, cotton, wool, viscose, polyamide, polyester, and/or polypropylene fibres or mixtures may be employed. Substrate type and specification are chosen in accordance with the end-use.

The invention imparts a characteristic that effectively kills a wide range of micro-organisms which come into contact with the treated material. It also prevents decomposition of the underlying substrate by inactivating or killing bacteria and other microbes on contact or in solution. The resultant treatment is partly or wholly resistant to degradation caused by most laundering and steam sterilisation processes. The treatment provides a characteristic coloration and some stiffening to the substrate, and improves the removal of organic staining when washed in a commercial laundry process.

It is known that atomic/metallic silver is a highly effective contact biocide, and chitosan is also known to exhibit the property of inactivating a wide range of micro-organisms which come into contact with it. It has now been found that atomic/metallic silver can be effectively dispersed through a polymeric structure of chitosan which itself has been deposited on the substrate. The dispersion is such that the silver substantially or wholly retains its biocidal properties. It has also been found that the system retains its biocidal properties when the chitosan is rendered insoluble and is fixed to the substrate. It has been shown that chemicals do not leach out from this system to any significant degree over the pH range of 5-9.

According to one aspect of the invention there is provided a process for treating a natural or synthetic substrate or blends thereof to impart biocidal properties to the substrate, said process comprising the steps of: (a) depositing or impregnating the substrate with solubilised chitosan, (b) immersing the substrate in a solution of silver salt, (c) treating the substrate to reduce the silver salt to atomic/metallic silver, (d) cross-linking the chitosan, and (e) washing and drying the resultant treated substrate.

The atomic/metallic silver is bound into the chitosan polymer by interaction with the amine groups of the latter, and is further entrapped by the cross-linking process which renders the chitosan insoluble. Silver is not easily removed from the system and represents a significant improvement on previous systems that merely utilise surface adsorption to provide a carrier for the silver. Such previous systems may also result in a "dusty" substrate surface, and a substrate that is difficult to handle and launder. The process of the present invention may be applied to substrates with a wide variety of end-uses, changing the substrate properties only marginally except with regard to anti-microbial properties.

The chitosan, which is required to have a minimum level of deacetylation sufficient to impart acid solubility, may be applied by deposition on, or impregnation of the substrate and rendered insoluble over a wide range of pH in conditions applicable to the use of surgical and similar garments, face masks, and health care dressings. The attachment of the chitosan to the substrate fibres is strong so that the resulting chitosan treatment can be regarded as very durable, often lasting the lifetime of the fabricated article and able to withstand commercial processes such as laundering and sterilisation.

Oxidative bleaching if required must be carried out on the substrate prior to treatment with chitosan. The treatment process imparts colour to the finished product which is characteristic of the process. Staining of articles in use is reduced due to the chemical nature of chitosan, which binds organic chromophores under acidic pH and releases them in alkaline conditions, as is used in commercial laundering processes. Stains are therefore washed from the treated fabric with greater ease than the untreated substrate.

The extent of treatment in respect to quantity of silver and chitosan to be deposited on the substrate is determined by the degree of biocidal effect required in the end product. It is also varied according to the nature of the substrate. Typically a deposition of 0.5- 3% of chitosan and 0.01 - 2% of silver salt by weight of fabric is suitable for end-use as garments and face masks.

Chitosan is solubilised by any of the methods common in the art, typically using a 2% w/w solution of acetic acid and may be applied to the substrate by padding, printing, spraying or pressure impregnation, and squeezed in a commercial mangle. The pressure of squeeze is adjusted to provide the amount of chitosan deposition required for ultimate performance of the end-use article. The chitosan is applied using one or several passes through the padder/mangle.

After neutralising the treated substrate with aqueous alkali such as caustic soda, it is immersed in a solution of silver salt, typically the nitrate, of strength to suit the ultimate end-use of the system, generally in the range 0.0005 - 2.0% w/v, preferably 0.001 - 0.2% w/v, and is then squeezed in a commercial mangle to remove excess liquid. Whilst silver nitrate is the preferred salt, any water soluble silver salt such as the acetate, perchlorate, difluoride, lactate or propionate may be used.

The neutralised substrate is then treated to reduce the silver salt to atomic/metallic silver by one of the methods common in the art, of which visible and/or ultraviolet light, gaseous hydrogen or hydroquinone are preferred for reasons of practicality.

The neutralised substrate can be bathed in an aqueous mixture of 0.2% w/v hydroquinone and 0.5% w/v sodium carbonate for up to 4 hours. Alternatively, one or more passes under a bank of commercial visible and/or ultraviolet light sources can be used to effect full conversion to atomic/metallic silver. The photochemical reduction process is made more efficient by a preliminary treatment in a 1% w/v solution of sodium chloride or a sodium chloride/sodium bromide mixture. If gaseous hydrogen is used the silver salt is first converted to the oxide using aqueous alkali before being reduced by hydrogen.

After washing with clean water the chitosan is cross-linked to render it insoluble at acidic pH. The cross-linking is performed using any of the methods common in the art, for instance by contacting the substrate with glutaraldehyde at a strength of 0.01 - 2.0% w/v for a period of 1-24 hours at room temperature. Alternatively, other dialdehydes or epichlorhydrin may be used.

The substrate is then washed with clean water and dried and is then ready to be fabricated into articles for end-use in medical and veterinary medicine such as face masks, dressings, surgical drapes, surgical gowns, protective clothing, as well as incontinence pads, sanitary towels, nappies, gloves, protective wrappings, sterile field and filters suitable for air, water or blood filtration.

Alternatively, the cross-linking process may be carried out before immersion in the silver salt solution. Here the chitosan-treated substrate is neutralised with aqueous alkali and the cross-linking process applied as described above.

After washing with clean water, the substrate is immersed in silver salt solution, squeezed and reduced as previously described. After washing with clean water, the substrate is dried and is ready for fabrication into end-uses.

Alternatively the cross-linking process may be modified to render the chitosan partially soluble at acidic pH. Here a low concentration of the cross-linking chemical is used, for instance glutaraldehyde, at a strength of 0.001 - 0.01% w/v, the amount being determined by reference to the quantity of chitosan deposited on the substrate and designed to cross-link a known proportion of the chitosan, for instance 50%. This enables a slow release of the silver and chitosan into the acidic aqueous phase while maintaining the contact biocide properties of the system itself.

In some instances it is possible to combine the steps of chitosan cross-linking with the silver salt treatment.

Generally, biocidal activity is diminished in use, not by significant use of the active ingredients (silver and chitosan), but by the accumulation of biomass which may be bound to the material, e.g. by a process similar to chelation. The material may be reactivated by washing at alkaline pH with clean water, and preferably with a cationic surfactant present. If the material has been in use under slightly or moderately alkaline conditions, washing at acidic pH and preferably with an anionic surfactant will effect reactivation wholly or in part.

The invention may be illustrated with reference to the following examples:

### EXAMPLE 1

### Method

Two samples of scoured 300g/m² cloth, weft 60 picks/inch (cotton) warp; 94 ends/inch (polyester) typically used for work-wear apparel, were treated with chitosan and silver and subjected to a microbial challenge test followed by a quantitative estimation of survivors in accordance with standard SNV 195 924 (Textile Fabrics: determination of the antibacterial activity, germ count method). (SNV 195 924 is a Swiss testing system protocol for textiles from Schweizerischen Normen-Vereinigung, Kirchen Veg 4, Postfach 8032, Zurich.)

### Treatment system

AT2/B - coated with 1.5%w/w of chitosan, neutralised and immersed in a 0.02%w/v solution of silver nitrate for 8 hours, washed with distilled water and placed in a bath of 1% sodium chloride for 1 hour. After washing, the cloth was cross-linked by immersing in 0.1w/v aqueous solution of glutyraldehyde for 8 hours and placed under a 20 watt 600 mm tube ultraviolet light source for 15 minutes and dried in air.

AT2/C - as sample AT2/B, with the sodium chloride step omitted.

### Microbial challenge

Samples were flash sterilised at 1.67 barg and 115°C for 1 minute and cut into circles 4.5cm diameter, three pieces per sample, and placed in screw-top jars which had also been previously flash sterilised to the same conditions.

An overnight culture of staphylococcus aureus NCTC 10788 was diluted aseptically in tryptone soya broth to give an approximate concentration of 10³ cells per ml.

1ml of this suspension was added to each of the samples, as far as possible covering the whole surface of the sample, the volume chosen to ensure no free liquor remained, i.e. all microbes were kept in contact with the samples.

The jars were incubated at 30°C for six hours whilst shaking at 100rpm on an orbital shaker.

### Testing for anti-microbial activity:

The samples were transferred aseptically to container of 10 ml of tryptone soya broth and vortexed vigorously. This process is intended to remove any bacteria from the fabric. The samples were removed as quickly as practical, squeezed in a clean empty dish, and placed firmly onto the surface of a fresh tryptone soya agar plate. Growth here would indicate the presence of live bacteria clinging to the sample. Results are shown in the table below "Beneath washed sample".

The broths vortexed from the samples were serially diluted down to 10⁻⁴ dilution and plate counts made of each dilution by the surface spread method. These counts give a quantitative estimation of cell numbers of live bacteria washed from the samples. Results shown in the table under "Plate count-washings".

These diluted broths were incubated for 24 hours at 37°C and examined by eye to determine presence or absence of turbidity. Turbidity indicates the survival of just one or two bacteria which may have been missed in the plate count of the diluted broths. Findings are reported in the table as "Incubated washings".

### Results

Results of microbiology are given in the following table, average values for three test pieces of each sample:

| | *Sample* | AT2/B | AT2/C |
|---|---|---|---|
| Plate count Washings | 10° | 0 | 0 |
| | 10⁻¹ | 0 | 0 |
| | 10⁻² | 0 | 0 |
| | 10⁻³ | 0 | 0 |
| | 10⁻⁴ | 0 | 0 |
| Incubated Washings | 10° | Growth | Growth |
| | 10⁻¹ | No growth | No growth |
| | 10⁻² | No growth | No growth |
| | 10⁻³ | No growth | No growth |
| | 10⁻⁴ | No growth | No growth |
| Beneath washed sample | | No growth | No growth |

### Conclusion

The results strongly indicate that samples AT2/B and AT2/C are virtually or probably 100% effective as biocides against staphylococcus aureus.

### EXAMPLE 2

### Method

Two samples of cloth of the same specification as in Example (1) were treated in the following manner:
AT3/B6 - coated with 1% w/w of chitosan, neutralised and cross-linked using 0.02%w/v aqueous solution of glutyraldehyde, washed with clean water and immersed in a 0.005%w/v solution of silver nitrate for 8 hours. It was then steeped for 60 minutes in a mixture of 0.4%w/v aqueous solution of sodium chloride and 0.4% w/v aqueous solution of sodium bromide. After washing the treated cloth was placed under a 20 watt 600 mm tube ultraviolet light source for 15 minutes and dried in air.
AT3/F10 - immersed in a 0.25%w/v solution of silver nitrate for 8 hours. It was then steeped for 60 minutes in a mixture of 0.4w/v aqueous solution of sodium chloride and 0.4%w/v aqueous solution of sodium bromide. After washing the treated cloth was placed under a 20 watt 600 mm tube ultraviolet light source for 15 minutes and dried in air. (i.e. chitosan coating and cross-linking steps omitted).

Both samples were washed in an automatic industrial laundry cycle using a commercial washing powder and dried in an industrial drier. Each sample was washed once at 60°C and twice at 95°C and dried.

The samples were tested using the strain of the micro-organism staphylococcus aureus designated NCTC 10788. The test protocol was the same as example (1), but dilutions were performed to 10⁻³. Results, averaged over three pieces for each sample, are given in the table below:

| *Sample Reference* | | AT3/B6 | AT3/F10* |
|---|---|---|---|
| *Treatment system* | | *Chitosan, X-linked* | |
| | | | |
| | | *Silver* | *Silver* |
| Plate count Washings | 10° | 0 | 31 |
| | 10⁻¹ | 0 | 9 |
| | 10⁻² | 0 | 2 |
| | 10⁻³ | 0 | 0 |
| Incubated Washings | 10° | No growth | Growth |
| | 10⁻¹ | No growth | Growth |
| | 10⁻² | No growth | No growth |
| | 10⁻³ | No growth | No growth |
| Beneath washed sample | | No growth | A few colonies on edges, no growth underneath. |

| | | | |
|---|---|---|---|
| * Not according to present invention | | | |

### Conclusions

Sample AT3/B6 indicates extremely good anti-microbial activity and sample AT3/F10 shows probable significant anti-bacterial activity. After laundering three times in severe conditions, the chitosan treatment system shows a higher degree of biocidal activity than the silver-only treatment.

### EXAMPLE 3

### Method

Samples of cloth described in example (1) were treated in the following ways and then tested in accordance with the SNV 195 924 protocol described in example (1) using the micro-organism staphylococcus aureus NCTC 10788. Results of these tests are summarised in the table, all results being the average for three test pieces per sample.

AT3/20 - this sample was not treated and used as a control.

AT2/D - 1%w/w of chitosan was padded on to the cloth, neutralised and then cross-linked in a 0.02%w/v aqueous solution of glutyraldehyde for 8 hours. The cloth was washed, dried in air and tested.

AT3/F18 - the cloth was immersed in a 0.005%w/v solution of silver nitrate for 8 hours. It was then steeped for 60 minutes in a mixture of 0.4%w/v aqueous solution of sodium chloride and 0.4%w/v aqueous solution of sodium bromide. After washing the treated cloth was placed under a 20 watt 600 mm tube ultraviolet light source for 15 minutes and dried in air and tested.

AT2/A - 1%w/w of chitosan was padded on to the cloth, neutralised and then cross-linked in a 0.02%w/v aqueous solution of glutyraldehyde for 8 hours. After rinsing with clean water the sample was then treated as AT3/F18 sample.

### Results

| *Sample Reference* | | AT3/20* | AT2/D* | AT3/F18* | AT2/A |
|---|---|---|---|---|---|
| *Treatment summary* | | *None* | *Chitosan* | | *Chitosan* |
| | | | | | |
| | | | *Cross-linked* | | *Cross-linked* |
| | | | | *Silver* | *Silver* |
| Plate count Washings | 10° | >300 | >300 | 33 | 0 |
| | 10⁻¹ | >300 | >300 | 8 | 0 |
| | 10⁻² | >300 | >300 | 2 | 0 |
| | 10⁻³ | 61 | >300 | 0 | 0 |
| | 10⁻⁴ | 32 | 256 | 0 | 0 |
| Incubated Washings | 10° | Growth | Growth | Growth | Growth |
| | 10⁻¹ | Growth | Growth | Growth | No growth |
| | 10⁻² | Growth | Growth | No growth | No growth |
| | 10⁻³ | Growth | Growth | No growth | No growth |
| | 10⁻⁴ | Growth | Growth | No growth | No growth |
| Beneath washed sample | | Massive Growth | Growth | No growth | No growth |

| | | | | | |
|---|---|---|---|---|---|
| *Not according to present invention | | | | | |

### Conclusions

Sample reference AT3/20 displayed an absence of anti-bacterial action. Sample AT2/D showed signs of inhibition, AT3/F18 is probably significantly biocidal and AT2/A is probably 100% effective biocidal activity. The combination of cross-linked chitosan with silver is more effective than silver or chitosan on their own.

### EXAMPLE 4

### Method

Two samples of the cloth referenced in example 3 were treated and tested to SNV 195 924 (see example (1) for details) using two micro-organisms, staphylococcus aureus NCTC 10788 and salmonella typhimurium strain NCTC 74.

Treatment: the samples were coated with 1% w/w of chitosan, neutralised and cross-linked using 0.02%w/w aqueous solution of glutyraldehyde for 8 hours, washed with clean water and immersed in a 0.005%w/v solution of silver nitrate for 1 hour. It was steeped for 5 minutes in a 1.0%w/v solution of sodium chloride. The treated cloth washed and was placed under a 20 watt 600 mm tube ultraviolet light source for 15 minutes and dried in air and tested.

Post treatment: the samples were laundered in an industrial washing cycle, once at 60°C and twice at 95°C and dried in a tumble drier.

### Results

| *Sample Reference* | | AT3/A9 | AT3/A9 |
|---|---|---|---|
| *Micro-organism* | | *Staph. Aureus* | *Salmonella typh.* |
| Plate count Washings | 10° | 2 | 35 |
| | 10⁻¹ | 0 | 6 |
| | 10⁻² | 0 | 0 |
| | 10⁻³ | 0 | 0 |
| Incubated Washings | 10° | No growth | No growth |
| | 10⁻¹ | No growth | No growth |
| | 10⁻² | No growth | No growth |
| | 10⁻³ | No growth | No growth |
| Beneath washed sample | | No growth | No growth |

### Conclusion

The samples are extremely biocidal to the micro-organisms used in the test.

### EXAMPLE 5

Samples of cotton cloth, 230 g/m² (weft 74 picks/inch, warp 56 ends/inch) were treated with either 0.8%w/w or 1.5%w/w chitosan and cross linked using 0.4%w/v glutyraldehyde for 12 hours, washed and neutralised. They were tested according to test method SNV 195 924 Textile Fabrics: determination of the antibacterial activity: germ count method.

Incubation temperatures were 37°C and samples were flash sterilised at the start of the test at 115°C and 1.67 barg pressure.

At the end of 6 and 12 hours, 100 ml of sterile distilled water containing 3% Tween 80 as neutraliser was added aseptically to the jars containing the samples, shaken vigorously for 1 minute and 1 ml aliquots removed for plating out.

Three micro-organisms were used in the tests:
methicillin resistant staphylococcus aureus strain SDRU R80/606
klebsiella pneumoniae strain NCIMB 10341
salmonella typhimurium strain NCTC 74

The log₁₀ CFU (colony forming units) figures were calculated and are tabulated below:

### Results

| Sample | MRSA | | | Kleb. Pneumoniae | | | Salmonella typh. | | |
|---|---|---|---|---|---|---|---|---|---|
| Control* | 5.81 | 9.05 | >10.5 | 6.16 | 9.13 | >10.5 | 6.33 | >9.5 | 10.11 |
| 0.8%* | 4.58 | 7.01 | 8.36 | 4.88 | 6.85 | 8.70 | 5.19 | 8.32 | 8.91 |
| 1.5%* | 4.94 | 6.93 | 7.85 | 4.99 | 7.16 | 8.96 | 5.01 | 9.25 | 9.32 |

| | | | | | | | | | |
|---|---|---|---|---|---|---|---|---|---|
| * Not according to present invention | | | | | | | | | |

### Conclusions

Both samples showed some growth inhibition of all the microbes tested, but do not pass the criteria for antibacterial action. The treatment system using chitosan alone is not effective as a contact biocide.

## Claims

1. A process for treating a natural or synthetic substrate or blends thereof to impart biocidal properties to the substrate, said process comprising the steps of:
(a) depositing or impregnating the substrate with solubilised chitosan,
(b) immersing the substrate in a solution of silver salt,
(c) treating the substrate to reduce the silver salt to atomic/metallic silver,
(d) cross-linking the chitosan, and
(e) washing the resultant treated substrate.

2. The process according to Claim 1, wherein the step of cross-linking the chitosan is carried out before the substrate is immersed in the silver salt solution.

3. The process according to Claim 1, wherein the steps of cross-linking the chitosan is carried out at the same time as the silver salt solution is applied to the substrate.

4. The process according to any one of Claims 1 to 3, wherein the substrate is comprised of fibres or yarn, is a non-woven, woven or knitted fabric or garment or a paper product.

5. The process according to Claim 4, wherein the fibres or yarn are cotton, wool, viscose, polyamide, polyester or polypropylene or mixtures thereof.

6. The process according to any one of Claims 1 to 4, wherein the treated substrate comprises from 0.5 to 3% by weight chitosan and from 0.01 to 2% by weight silver salt.

7. The process according to any one of Claims 1 to 6, wherein the chitosan is solubilised in dilute acid.

8. The process according to Claim 7, wherein after treating the substrate with chitosan and before immersing said substrate in the silver salt solution the chitosan-treated substrate is neutralised with aqueous alkali.

9. The process according to any one of Claim 1 to 8,wherein the solubilised chitosan is applied to the substrate by padding, printing, spraying or pressure impregnation.

10. The process according to any one of Claims 1 to 9, wherein the solution of silver salt is a solution of silver nitrate or any water soluble silver salt such as the acetate, perchlorate, difluoride, lactate, or propionate salt.

11. The process according to Claim 10, wherein the concentration of silver nitrate solution is from 0.0005 to 2.0% w/v.

12. The process according to Claim 11, wherein the concentration of silver nitrate solution is from 0.001 to 0.2% w/v.

13. The process according to any one of Claims 1 to 12, wherein the silver salt is converted to the oxide by aqueous alkali and reduced by gaseous hydrogen.

14. The process according to any one of Claims 1 to 12, wherein the silver salt is reduced by visible and/or ultraviolet light.

15. The process according to Claim 14, wherein the substrate is treated with a solution of dilute sodium chloride or a sodium chloride/sodium bromide mixture prior to reducing the silver salt by visible and/or ultraviolet light.

16. The process according to any one of Claims 1 to 12, wherein the silver salt is reduced by hydroquinone.

17. The process according to Claim 16, wherein the silver salt is reduced using an aqueous mixture of 0.2% w/v hydroquinone and 0.5% w/v sodium carbonate.

18. The process according to any one of Claims 1 to 17, wherein the chitosan is cross-linked by contacting the chitosan-treated substrate with an aqueous solution of glutaraldehyde, or other dialdehyde or epichlorhydrin.

19. The process according to Claim 18, wherein the concentration of glutaraldehyde is from 0.001 to 0.1% w/v.

20. The process according to Claim 18, wherein the concentration of glutaraldehyde is from 0.01 to 2.0% w/v.

21. The process according to any one of Claims 18 to 20, wherein the chitosan-treated substrate is contacted with glutaraldehyde for a period of 1 to 24 hours at room temperature.

## Patentansprüche

1. Verfahren zur Behandlung eines natürlichen oder synthetischen Trägermaterials oder Mischungen davon, um dem Trägermaterial Biozideigenschaften zu geben, wobei besagtes Verfahren folgende Schritte umfasst:
(a) Ablagerung von Chitosan auf das Trägermaterial bzw. Imprägnieren des Trägermaterials mit Chitosan
(b) Eintauchen des Trägermaterials in eine Silbersalzlösung
c) Behandeln des Trägermaterials, um das Silbersalz auf atomares/metallisches Silber zu reduzieren,
(d) Quervernetzen des Chitosans, und
(e) Waschen des resultierenden, behandelten Trägermaterials.

2. Verfahren nach Anspruch 1, wobei der Schritt der Quervernetzung des Chitosans vor dem Eintauchen des Trägermaterials in die Silbersalzlösung ausgeführt wird.

3. Verfahren nach Anspruch 1, wobei der Schritt der Quervernetzung des Chitosans zur selben Zeit wie das Aufbringen der Silbersalzlösung auf das Trägermaterial ausgeführt wird.

4. Verfahren nach einem der Ansprüche 1 bis 3, wobei das Trägermaterial aus Fasern oder Garn besteht, ein Vliesstoff, eine Web- oder Wirkware oder Kleidungsstück oder ein Papierprodukt ist.

5. Verfahren nach Anspruch 4, wobei die Fasern oder das Garn aus Baumwolle, Wolle, Viskose, Polyamid, Polyester oder Polypropylen oder Mischungen daraus sind.

6. Verfahren nach einem der Ansprüche 1 bis 4, wobei das behandelte Trägermaterial 0,5 bis 3 Gewichts-% Chitosan und von 0,01 bis 2 Gewichts-% Silbersalz umfasst.

7. Verfahren nach einem der Ansprüche 1 bis 6, wobei das Chitosan in verdünnter Säure löslich gemacht wird.

8. Verfahren nach Anspruch 7, wobei, nach der Behandlung des Trägermaterials mit Chitosan und vor dem Eintauchen des besagten Trägermaterials in die Silbersalzlösung, das mit Chitosan behandelte Trägermaterial mit wässerigem Alkali neutralisiert wird.

9. Verfahren nach einem der Ansprüche 1 bis 8 , wobei das löslich gemachte Chitosan durch Klotzen, Drucken, Sprühen oder Druckimprägnierung auf das Trägermaterial aufgebracht wird.

10. Verfahren nach einem der Ansprüche 1 bis 9, wobei die Lösung des Silbersalzes eine Lösung aus Silbernitrat oder ein beliebiges wasserlösliches Silbersalz, wie beispielsweise das Acetat, Perchlorat, Difluorid, Lactat oder Propionatsalz, ist.

11. Verfahren nach Anspruch 10, wobei die Konzentration der Silbernitratlösung von 0,0005 bis 2,0% w/v (Gewicht zu Volumen) reicht.

12. Verfahren nach Anspruch 11, wobei die Konzentration der Silbernitratlösung von 0,0001 bis 0,2% w/v (Gewicht zu Volumen) reicht.

13. Verfahren nach einem der Ansprüche 1 bis 12, wobei das Silbersalz durch wässeriges Alkali zum Oxid umgewandelt und durch gasförmigen Wasserstoff reduziert wird.

14. Verfahren nach einem der Ansprüche 1 bis 12, wobei das Silbersalz durch sichtbares und/oder ultraviolettes Licht reduziert wird.

15. Verfahren nach Anspruch 14, wobei das Trägermaterial, vor dem Reduzieren des Silbersalzes durch sichtbares und/oder ultraviolettes Licht, mit einer Lösung aus verdünntem Natriumchlorid oder einer Natriumchlorid/Natriumbromidmischung behandelt wird.

16. Verfahren nach einem der Ansprüche 1 bis 12, wobei das Silbersalz durch Hydrochinon reduziert wird.

17. Verfahren nach Anspruch 16, wobei das Silbersalz durch Verwenden einer wässerigen Mischung von 0,2% w/v (Gewicht zu Volumen) Hydrochinon und 0,5% w/v (Gewicht zu Volumen) Natriumcarbonat reduziert wird.

18. Verfahren nach einem der Ansprüche 1 bis 17, wobei das Chitosan durch in Berührung bringen des mit Chitosan behandelten Trägermaterials mit einer wässerigen Lösung von Glutaraldehyd oder einem anderen Dialdehyd oder Epichlorhydrin vernetzt wird.

19. Verfahren nach Anspruch 18, wobei die Konzentration des Glutaraldehyd von 0,001 bis 0,1% w/v (Gewicht zu Volumen) reicht.

20. Verfahren nach Anspruch 18, wobei die Konzentration des Glutaraldehyd von 0,01 bis 2% w/v (Gewicht zu Volumen) reicht.

21. Verfahren nach einem der Ansprüche 18 bis 20, wobei das mit Chitosan behandelte Trägermaterial für einen Zeitraum von 1 bis 24 Stunden bei Raumtemperatur mit Glutaraldehyd in Berührung gebracht wird.

## Revendications

1. Un procédé pour traiter un substrat naturel ou synthétique ou leurs mélanges pour communiquer des propriétés biocides au substrat, ce procédé comprenant les étapes suivantes:
a) le dépôt ou l'imprégnation du substrat avec du chitosan
b) l'immersion du substrat dans une solution de sel d'argent
c) le traitement du substrat pour réduire le sel d'argent en argent atomique/métallique
d) réticulant le chitosan, et
e) lavant le substrat traité qui en résulte.

2. Le procédé selon la revendication 1, dans lequel l'étape de réticulation du chitosan est effectuée avant que le substrat soit immergé dans la solution de sel d'argent.

3. Le procédé selon la revendication 1, dans lequel les étapes de réticulation du chitosan sont effectuées en même temps que l'application de la solution de sel d'argent au substrat.

4. Le procédé selon l'une quelconque des revendications 1 à 3, dans lequel le substrat est composé de fibres ou de fil, et où il est une étoffe ou un vêtement tissé, non-tissé ou tricoté ou un produit en papier.

5. Le procédé selon la revendication 4, dans lequel les fibres ou le fil sont en coton, en laine, en viscose, en polyamide, polyester ou polypropylène ou en mélanges de ceux-ci.

6. Le procédé selon l'une quelconque des revendications 1 à 4 dans lequel le substrat traité comprend de 0,5 à 3% en poids de chitosan et de 0,01 à 2% en poids de sel d'argent.

7. Le procédé selon l'une quelconque des revendications 1 à 6, dans lequel le chitosan est solubilisé dans de l'acide dilué.

8. Le procédé selon la revendication 7, dans lequel, après le traitement du substrat au chitosan et avant immersion du substrat dans la solution de sel d'argent, le substrat une fois traité au chitosan est neutralisé à l'aide d'alcali aqueux.

9. Le procédé selon l'une des revendications 1 à 8, dans lequel le chitosan solubilisé est appliqué au substrat par immersion, impression, pulvérisation ou par imprégnation sous pression.

10. Le procédé selon l'une quelconque des revendications 1 à 9, dans lequel la solution de sel d'argent est une solution de nitrate d'argent ou tout autre sel d'argent soluble à l'eau tel que l'acétate, le perchlorate, le difluorure, le lactate ou le propionate de sel.

11. Le procédé selon la revendication 10, dans lequel la concentration de solution de nitrate d'argent est de 0,0005 à 2,0% w/v.

12. Le procédé selon la revendication 11, dans lequel la concentration de la solution de nitrate d'argent est de 0,001 à 0,2% w/v.

13. Le procédé selon l'une quelconque des revendications 1 à 12, dans lequel le sel d'argent est converti en oxyde d'argent par l'alcali aqueux et réduit par de l'hydrogène gazeux.

14. Le procédé selon l'une quelconque des revendications 1 à 12, dans lequel le sel d'argent est réduit par de la lumière visible et/ou ultraviolette.

15. Le procédé selon la revendication 14, dans lequel le substrat est traité avec une solution de chlorure de sodium dilué ou un mélange de chlorure de sodium/bromure de sodium avant de réduire le sel d'argent par la lumière visible et/ou par lumière ultraviolette.

16. Le procédé selon l'une quelconque des revendications 1 à 12, dans lequel le sel d'argent est réduit par hydroquinone.

17. Le procédé de la revendication 16, dans lequel le sel d'argent est réduit en utilisant un mélange aqueux de 0,2% w/v d'hydroquinone et 0,5% w/v de carbonate de sodium.

18. Le procédé selon l'une quelconque des revendications 1 à 17, dans lequel le chitosan est réticulé en exposant le substrat traité au chitosan à une solution aqueuse de glutaraldéhyde ou autre dialdéhyde ou épichlorhydrine.

19. Le procédé selon la revendication 18, dans lequel la concentration de glutaraldéhyde est de 0,001 à 0,1% w/v.

20. Le procédé selon la revendication 18, dans lequel la concentration de glutaraldéhyde est de 0,01 à 2,0% w/v.

21. Le procédé selon l'une quelconque des revendications 18 à 20, dans lequel le substrat traité au chitosan est exposé au glutaraldéhyde pendant une période de 1 à 24 heures à température ambiante.
